# EUROPEAN PATENT SPECIFICATION

(11) **EP 3 074 344 B1**
(45) Date of publication and mention of the grant of the patent: **12.02.2020**
(21) Application number: 14793454.1
(22) Date of filing: 31.10.2014
(51) Int. Cl.: A61K 9/00, A61K 9/50, A61K 31/216, C01B 33/18, C01B 33/143, A61K 47/02, C01B 33/193, A61K 9/14

(54) **PROCESS FOR PRODUCING INORGANIC PARTICULATE MATERIAL**
VERFAHREN ZUR HERSTELLUNG ANORGANISCHER PARTIKEL
PROCÉDÉ DE PRODUCTION DE MATÉRIAU PARTICULAIRE INORGANIQUE

(30) Priority: 26.11.2013 EP 13005505; 06.12.2013 EP 13005688
(43) Date of publication of application: 05.10.2016
(73) Proprietor: Merck Patent GmbH, 64293 Darmstadt (DE)
(72) Inventor: KUCERA, Shawn, Cedar Park, TX 78613 (US); LUBDA, Dieter, 64625 Bensheim (DE); FRIEDRICH, Lars, 64385 Reichelsheim (DE); WITT, Vanessa, 64739 Hoechst (DE)
(86) International application number: PCT/EP2014/002929
(87) International publication number: WO 2015/078552

(56) References cited:
- WO-A1-2012/156023
- WO-A1-2014/072015
- BOFFA ET AL: "Preparation of templated mesoporous silica membranes on macroporous alpha-alumina supports via direct coating of thixotropic polymeric sols", MICROPOROUS AND MESOPOROUS MATERIALS, ELSEVIER SCIENCE PUBLISHING, NEW YORK, US, vol. 100, no. 1-3, 7 February 2007 (2007-02-07), pages 173-182, XP005878673, ISSN: 1387-1811, DOI: 10.1016/J.MICROMESO.2006.10.035
- ANA GARCA ET AL: "Preparation of 3-D scaffolds in the SiOPOsystem with tailored hierarchical meso-macroporosity", ACTA BIOMATERIALIA, ELSEVIER, AMSTERDAM, NL, vol. 7, no. 3, 11 October 2010 (2010-10-11), pages 1265-1273, XP028131218, ISSN: 1742-7061, DOI: 10.1016/J.ACTBIO.2010.10.006 [retrieved on 2010-10-15]
- SCHUTH ET AL: "Superstructures of mesoporous silicas", CURRENT OPINION IN COLLOID AND INTERFACE SCIENCE, LONDON, GB, vol. 3, no. 2, 1 April 1998 (1998-04-01), pages 174-180, XP027090756, ISSN: 1359-0294 [retrieved on 1998-04-01]

## Description

The present invention relates to a process for producing inorganic particulate material.

Inorganic materials are receiving a great interest in the field of biomedical science in the last few years. Two main routes have been traditionally used for drug intake: oral administration and injection. Traditional therapies are characterized by an increase of drug concentration in plasma when the intake takes place, followed by a decrease, leading to a sinusoidal behavior of the drug concentration in plasma vs. time.
Inorganic materials, especially bioceramics, have some porosity that can be used for drug delivery including chemically synthesized substances such as, for example, ibuprofen or nimodipine, but also biologically derived substances such as, for example, releasing growth factors or proteins. Especially silica-based ordered mesoporous materials are possible candidates as reservoir bioceramics where drugs can be confined.
These materials are characterized by large specific surface areas, ordered pore systems, and narrow pore size distributions. In addition, these mesoporous materials have been reported to be excellent candidates to be used in tissue engineering nanotechnology, because they show the capability to perform as controlled delivery systems of a wide range of drugs and to promote bone tissue regeneration.
Depending of their pore size inorganic materials can be classified as microporous, mesoporous or macroporous. Within the meaning of the present application microporous materials are understood to have a pore size < 2 nm, mesoporous materials are understood to have a pore size from 2 to 100 nm and macroporous materials are understood to have a pore size > 100 nm.
In recent years ordered porous materials have been increasingly studied for the use as drug delivery systems. From such materials mesoporous silica has been of specific interest.

One main approach for using mesoporous silica for the formulation of drug delivery systems is to increase the dissolution rate of poorly water-soluble or water-insoluble active pharmaceutical ingredients. Poorly water-soluble or insoluble active pharmaceutical ingredients usually have a very low bioavailability due to their poor solubility in digestive fluids causing incomplete absorption. The rationale of using mesoporous silica for use in drug delivery systems is to increase the dissolution rate of poorly water-soluble or water-insoluble active pharmaceutical ingredients and thereby to improve their bioavailability.
Ordered mesoporous materials, which have been extensively studied, are e.g. MCM-41 (Mobil Composition of Matter number forty one) and SBA-15 (Santa Barbara Amorphous number fifteen). SBA-15 was first described by Zhao et al. and is the result of a templating procedure based on a hexagonal arrangement of amphiphilic block copolymers (D.Y. Zhao et al.: Triblock copolymer syntheses of mesoporous silica with periodic 50 to 300 angstrom pores, Science 279 (1998) 548-552). MCM-41 is obtained by the template action of long chain alkylammonium surfactant molecules (J.S. Beck et al.: A new family of mesoporous molecular sieves prepared with liquid-crystal templates, J. Am. Chem. Soc. 114 (1992) 10834-10843). Typically, the pore diameter varies between 2 and 6 nm for MCM-41 and between 4 and 13 nm for SBA-15. In addition to the well-defined mesopore system, SBA-15 has a complementary pore system comprised of micropores (pore size < 2 nm). These micropores are located in the walls between adjacent mesopores and do not bridge the wall; they constitute dead end pores (J.S. Beck et al.: A new family of mesoporousmolecular sieves prepared with liquid-crystal templates, J. Am. Chem. Soc. 114 (1992) 10834-10843).
Vallet-Regi et al. were one of the first to explore the drug release properties of these materials in an attempt to prolong the release of ibuprofen using MCM-41 as a carrier (M. Vallet-Regi et al.: A new property of MCM-41: drug delivery system, Chem. Mater. 13 (2001) 308-311). The release kinetics of drugs from mesoporous silica carriers is dependent on several material characteristics including pore size (P. Horcajada et al.: Influence of pore size of MCM-41 matrices on drug delivery rate, Microporous Mesoporous Mater. 68 (2004) 105-109), pore connectivity (J. Andersson et al.: Influences of material characteristics on ibuprofen drug loading and release profiles from ordered micro- and mesoporous silica matrices, Chem. Mater. 16 (2004) 4160-4167) and the chemical composition of the silica surface (B. Munoz et al.: MCM-41 organic modification as drug delivery rate regulator, Chem. Mater. 15 (2003) 500-503).
WO 2006/026840 A2 discloses a controlled release delivery system wherein amorphous mesoporous non-fibrous silica is used as matrix carrier for the release of bioactive compounds and wherein such matrix carrier further comprises micropores having a mean size in the range of 0.4 to 2.0 nm.
WO 2005/000740 A2 discloses a crystalline mesoporous silica material comprising a framework of zeolite type micropores (designated as nanometer size building units), which does not give rise in Bragg type diffraction in x-ray diffraction, and its use for drug delivery.
Z.G. Shi et al. describe mesoporous silica particles for drug delivery, which beside the mesopores further contain macropores (Z.G. Shi et al.: Drug delivery devices based on macroporous silica spheres, Micropor. Mesopor. Mater. 126 (2011) 826-831). Due to its penetrable macropores the mesopores of such material can be sufficiently and efficiently loaded with drug.
The silica particles described by said publication from Z.G. Shi et al. are produced by using sol-gel technique in combination with an emulsion method and phase separation as described by Z.G. Shi et al. in 2008 (Z.G. Shi et al.: Synthesis and characterization of hierarchically porous silica microspheres with penetrable macropores and tunable mesopores, Micropor. Mesopor. Mater. 116 (2008) 701). In brief a solution containing tetraethoxyorthosilicate (TEOS), polyethylene oxide and hydrochloric acid are mixed and stirred and the ethanol resulting from the hydrolization of TEOS is removed by vacuum pumping for 4 h. Then the resulting solution is dispersed into paraffin oil under vigorous stirring. 20 hours later the resulting product was repeatedly washed with ethanol and water and subsequently calcined for 2 h at 600°C. The obtained calcined silica was size classified by using liquid elutriation involving the steps dispersion of the silica particles into water by ultrasonic treatment for 5 min., subsiding the particles in the dispersion by keeping it static for 2 hours, and discarding the upper water solution containing the small particles. Such size-classification was repeated for five times and the particles were collected.

WO 2012/156023 A1 describes a method for producing porous silica material containing both macropores and mesopores by using the sol-gel technique, as well as the use of the material in delivering a biologically active agent. In brief, the synthesis is carried out by dissolving a water-soluble polymer or another pore forming agent and a precursor for a matrix dissolving agent in a medium that promotes the hydrolysis of the organometallic compound. The organometallic compound, or a mixture of organometallic which contains hydrolysable ligands to promote a hydrolysis reaction, is mixed. The mixture is then solidified through a sol-gel transition, whereby a gel is prepared which has three-dimensional interconnected phase domains: one rich in solvent and the other rich in inorganic component in which surface pores are contained. The gel is then disintegrated into particles by agitation. The next step involves setting the matrix dissolving agent free from its precursor, whereby the matrix dissolving agent modifies the structure of said inorganic component. This is followed by removing the solvent by evaporative drying and/or heat treatment. Finally, the particles are calcined to form the porous material.

In the state of the art particle size and distribution are controlled by adjusting the time period from phase separation until stirring. Before and during this time period the reaction mixture is not stirred. Particle formation having a small size and a narrow particle size distribution is promoted by short time periods from phase separation until stirring, whereas increased particle sizes is promoted by increasing the time period from phase separation until stirring. Therefore, the time point of "phase separation" of the reaction mixture is critical to observe. The phase separation is characterized by a characteristic light blue "haze". Until this point there has been no suitable means to measure the occurrence of the phase separation. It has always been the responsibility of the scientist performing the synthesis to optically determine the point of phase separation. Because this is a human measurement, it is prone to variability between operators viewing the reaction and also can be affected by variables such as lighting, background, and experience. The phase separation occurs according to a spinodal decomposition mechanism, which is essentially a rapid unmixing of liquids or solids from one thermodynamic phase to form two coexisting phases. In this sense, the silica precursor is separating from a homogenous mixture with the aqueous media and polymerizing to form one phase rich in silica and a second aqueous phase.

The object of the present invention was therefore to provide a process for producing inorganic particulate material assuring that the reaction conditions may be maintained between batches and that the size distribution of the resulting particles can be selected and adjusted easily.

Surprisingly, it was found that stirring during the entire reaction allows for a better control of the reaction conditions and of the resulting particle size.

In a first aspect the present invention is therefore directed to a process for producing inorganic particulate material mainly composed of silicon oxide, wherein the particulate material comprises mesopores and macropores and the process includes the steps of:
(a) dissolving a water-soluble polymer or another pore forming agent and a precursor for a matrix dissolving agent in a medium that promotes the hydrolysis of the metalorganic compound (see step b);
(b) mixing a metalorganic compound or a mixture of metalorganic compounds which contains hydrolyzable ligands to promote hydrolysis reaction;
(c) solidifying the mixture through a sol-gel transition, wherein the mixture is continuously stirred, whereby particles are prepared which have three dimensional interconnected phase domains one rich in solvent the other rich in inorganic component in which surface pores are contained, wherein in step (c) changes in torque are monitored;
(d) setting the matrix dissolving agent free from its precursor, whereby the matrix dissolving agent modifies the structure of said inorganic component;
(e) removing the solvent by evaporation drying and/or heat-treatment.

As a prejudice in the state of the art it was assumed that stirring in the reactor must be discontinued in order to allow the silica gel to form. Surprisingly, it was found that stirring during the time leading to the phase separation does not negatively influence the material with respect to its physical and chemical properties.
In addition, stirring during the entire reaction allows for a better control of the reaction conditions and the resulting particle size: As the silica network begins to form during the phase separation, there is an increase in viscosity of the reaction mixture due to the formation of the silica gel. If the mixture is continually stirred during the entire reaction, the increase in viscosity is reflected on the drive motor as an increase in torque. This means of production is advantageous in that by carrying out the synthesis after phase separation according to changes in torque, it can be assured that the reaction conditions will be maintained between batches. Therefore, this production process is independent of operator influence, or error, in determining the point at which phase separation begins and offers a simple method to quantify at what point in time after phase separation the mixing rate should be increased to produce particles having a certain uniform size distribution so that future size classification steps can be avoided.

The present invention is therefore a process as described above, wherein in step (c) changes in torque are monitored.

In a preferred embodiment of the process according to the present invention in step (c) the size of the particles is controlled by adjusting the stirring speed over the reaction time.

In the process according to the present invention the size of the particles may be controlled by adjusting the stirring speed over the reaction time by monitoring changes in torque.

In another embodiment of the present invention as defined above, the time point of phase separation may be determined by monitoring changes in torque.

In a further embodiment of the present invention the reaction time may be determined by monitoring changes in torque.

The stirring in step (c) can be performed, for example, with an agitator, a high shear mixer (e.g. Ultraturrax®), ultrasonics, or by an overhead stirrer (e.g. Heidolph Rührwerk RZR 2102 Control).
Preferably, the gel is transferred and homogenized to a particulate material by the use of an overhead stirrer. The particle size and distribution can be controlled by selecting the conditions of the process, especially by monitoring the torque value and adjusting the stirring speed, and the time point when the stirring speed is changed. In principle to achieve smaller particle sizes, the mixing/stirring speed is increased at a lower torque value (i.e. earlier in the reaction process), while the opposite is true to achieve larger particle sizes (i.e. increase of stirring speed at a higher torque value and later in the reaction process).

Surprisingly it has been found that very uniform particle distributions can be obtained by controlling such parameters. Therefore, the process of the invention further offers a simple method to produce particles having a uniform size distribution so that successive classification steps as described in the prior art can be avoided.

The stirring device/ agitator can be any suitable device known in the art. It can be one where the angle of the blades providing agitation are bent at an angle of 0° to 180° relative to the horizontal surface of the vessel; preferably from 0° to 90°; more preferably from 30° to 90°; most preferably from 45° to 90°. The agitating device can consist of a vertical shaft composed of one set of stirring blades or a number of stirring blades attached to the same shaft. The stirring blades can be configured so that all are of the same angle, or configured that the blades are of various angles. Stirring devices as such are known to a person skilled in the art, e.g. an overhead stirrer (Heidolph Rührwerk RZR 2102 Control). Preferably, the stirring device has a built-in torque measurement function.

According to the present invention the point in time after phase separation when the mixing speed should be increased to produce particles having a uniform size distribution may be determined by monitoring the torque value. Torque can be described as the tendency of a force to rotate an object about an axis, fulcrum, or pivot. Torque is also known as "moment of force". As resistance to rotation is increased, the power needed by a force to rotate the object at a set rate increases. Thus, as the viscosity of the sol-gel increases due to phase separation of the reaction mixture, the power needed by the overhead stirrer to turn the mixing blades at the same RPM increases and can be viewed as an increase in torque.

For example, typical torque values for a 1L-scale at the time point when the mixing speed is increased can be in the range of 2.0 to 4.5 Ncm (relative to the torque value prior to the start of the reaction, i.e. before step (a)). For example, if the torque value is 2.0 Ncm, a material having a mean particle size of about 7.6 µm and a narrow particle size distribution (d10 - 3.7 µm, d50 - 7.6 µm, d90 - 18.0 µm) is obtained. If the torque value is 4.5 Ncm, a material having a mean particle size of 18.0 µm and a broader particle size distribution (d10 - 5.4 µm, d50 - 18.0 µm, d90 - 42.6 µm) is obtained. Changes in stirring blade geometry, as well as reactor size and design, could result in different torque values; however, this is not always the case. For example, in a larger reactor the torque value at the time point when the mixing speed should be increased is 5.3 Ncm.

The torque values can be monitored by any method common to a person skilled in the art. Preferably, the stirring device has a built-in torque measurement function, so that the torque value can easily be observed during the process, e.g. via a digital display. In practice, the torque value can, for example, be zeroed prior to the start of the reaction (i.e. before step (a)). The relative change in torque can then easily be observed during the reaction. Many common stirring devices include means for measuring torque values. In this case the torque values can, for example, easily be monitored via a display of the device.

In a preferred embodiment of the invention the process as set forth above comprising the steps (a) to (e) further comprises the step (f) calcining the particles.

The process of the invention typically leads to particulate material having a mean diameter from about 1 µm to about 2000 µm, preferably from about 1 µm to 1000 µm and more preferably from about 1 µm to 500 µm
Advantageously all steps of the process can be performed in the same reaction vessel. Preferably the vessel used for the process is a closable one, which allows the formation of a saturated vapor pressure, so that the liberation of the matrix dissolving agent from its precursor can be performed in an easy and time efficient manner as described later on.

In the process of the present invention a water-soluble polymer suitable to induce pore formation by a phase separation process or other pore forming agents is used to control porosity of the material. The pore forming agents have considerable solubility in water and water-alcohol mixed solvents and have to be uniformly dissolved in the solvent mixture generated during the hydrolysis reaction of metalorganic compound containing hydrolyzable ligands. Pore forming agents which can be used as part of the pore forming phase in producing the porous particles according to the invention are desired to have considerable solubility in water and water-alcohol mixed solvents. They have to be uniformly dissolved in the solvent mixture generated during the hydrolysis reaction of the metalorganic compound containing hydrolyzable ligands, such as, for example, silicon alkoxide.

Water-soluble polymers suitable to induce pore formation are, for example, polymeric salts such as poly(sodium styrenesulfonate) or poly(potassium styrenesulfonate), polymeric acids which may dissociate to become polyanion such as poly(acrylic acid), polymeric bases which may dissociate to become polycation such as poly(allylamine) or poly(ethyleneimine), non-ionic polymers having ether oxygen in the main chain such as poly(ethylene oxide), non-ionic polymers having lactone units in the side chain such as poly(vinylpyrrolidone) are suitable examples. Preferred polymers are non-ionic surfactants such as ether derivatives of polyoxyethylene, especially those containing an alkyl-, aryl-, alkylaryl- (e.g. an alkylphenyl), or arylalkyl (e.g. phenylalkyl) residue. Non-ionic surfactants possessing polyoxypropylene residues as hydrophilic moiety, such as polyoxypropylene alkyl ethers can also be used. Preferred polyethylene oxide containing surfactants are those which are derivatized with a lipophilic alkyl group with 8 to 20 C atoms, or with a lipophilic aryl group which can be substituted with one or several alkyl groups, and which have 6 to 25 C-atoms in total.

Examples of the latter group of polyethylene oxide containing surfactants are polyoxyethylene nonylphenyl ether, polyoxyethylene octylphenyl ether, or polyoxyethylene (1,1,3,3-tetramethylbutyl)-phenyl ether. However, these examples are not limitative.

The hydrophilic lipophilic balance (HLB) system can be used to estimate the behavior of nonionic surfactants, and can be used as a guideline for exchanging different non-ionic surfactants. The amount of non-ionic surfactant to be added varies, depending on the type of said non-ionic surfactant and also on the type and the amount of the metal alkoxide added, but may be from 1.0 to 10.0 g, preferably from 1.5 to 6.0 g, per 10 g of the metal alkoxide.

The non-ionic surfactant has the function of inducing both sol-gel conversion and phase separation at the same time. While being gelled, the reaction system is separated into a solvent-rich phase and a silica rich phase. According to a preferred embodiment of the invention is directed to the process as described, wherein the pore forming agent is the non-ionic surfactant.

Metalorganic compound can be applied by hydrolyzing metal alkoxides, metal chlorides, metal salts or coordinated compounds. In this process an organic polymer is used, which is compatible with the solution of the metal alkoxide or its polymer, and which undergoes phase separation during the hydrolysis-polymerization step. This method comprises preparing a gel which has a solvent-rich phase capable of giving macropores of not smaller than about 100 nanometers in size, through sol-gel conversion in the presence of a pore forming agent, and finally drying and calcining the material. The porous inorganic particles produced by this process display connected open macropores. Examples of pore forming agents disclosed in these documents are: Adding lower alkyl alcohols like methanol or ethanol to the gelling mixture can also be used to modify the size of the macropores. In the present invention the sol-gel method is used to control the pore size of the porous inorganic particles.

Metalorganic compounds having a hydrolysable ligand like metal alkoxides are used as starting materials with additions of appropriate chemical substances to result in the formation of characteristic phase-separated structure of which solvent-rich pore forming phase converts to the macropore of the dried gel material: Such starting materials and the conditions necessary to hydrolyse these starting materials are known in the art. Preferred metal alkoxides are silicon alkoxides, which may include, for example, tetramethoxysilane (TMOS), tetraethoxysilane (TEOS), methyltrimethoxysilane, ethyltrimethoxysilane and vinyl trimethoxysilane. However, these examples are not limitative. Other suitable metal alkoxides or other suitable metal compounds including mixtures of these compounds are known in the art.

The conditions are chosen so as to hydrolyze the metal compound having a hydrolyzable functional group and to cause polymerization of the metal compound. At the same time the sol-gel transition of the reacting solution and the phase separation into solvent rich phase and a phase rich in metal compound (skeleton phase) are induced. For silicon alkoxides as metal compound having a hydrolyzable functional group the hydrolysis is done in an acidic medium. Diluted organic or inorganic acids are preferred in this case. Especially preferred is the use of acetic acid, hydrochloric acid or nitric acid using concentrations between 1 mmol/I and 2 mol/l. Other acidic media suitable to carry out the hydrolysis of silicon alkoxides are known in the art. Suitable reagents for the hydrolysis of other metalorganic compounds are known in the art as well.

The process of the present invention further includes a precursor, i.e. a precursor for a matrix dissolving agent, which is used to control mesoporosity of the material. Use of a precursor allows its addition from the beginning so that the precursor is and remains dissolved during sol-gel transformation. The liberation of the matrix dissolving agent can be induced later on, for example by heating, which leads to liberation of the matrix dissolving agent by chemical decomposition (thermolysis). Matrix dissolving agents to be liberated from the precursor are basic substances such as ammonia. Precursors which can used in the present invention to liberate ammonia are, for example, urea and organic amides such as formamide, N-methylformamide, N,N,-dimethylformamide, acetamide, N-methylacetamide, and N,N-dimethylacetamide. Preferred precursors are compounds having an amido group or an alkyl amido group, especially preferred is urea. Accordingly, one embodiment of the invention is directed to the process for producing the inorganic particulate material, wherein said precursor of the matrix dissolving agent is a compound having an amido group or an alkylamido group, preferably urea.

The amount of the thermolyzable compound in the reaction system of the present invention may vary, depending on the type of said compound. Urea, for example, may be used in an amount from 0.1 to 3 g, preferably from 0.2 to 2 g, per 10 g of the reaction system (reaction system = sum of all ingredients). The heating temperature for the thermolysis of urea may fall between 60°C and 200°C. It is preferred that the thermolyzing step is executed in a closed container in order to make the vapor pressure of the thermolyzed product saturated and to rapidly make the solvent have a steady pH-value. After the thermolysis, the pH of the solvent is preferably from 8.0 to 11.0. The time after which the pore structure of the gel stays substantially unchanged under the processing conditions depends on the type of the precursor for the matrix dissolving agent and on the conditions applied (e.g. the temperature); when urea is used as the precursor for the matrix dissolving agent the necessary time typically is between 30 minutes (e.g. at 200 °C) and 30 days (e.g. at 60°C). Preferably the gel is treated with urea at 110°C for about 4 hours which leads to a mesoporous material with ca. 10-13 nm pore size.

The final preparation steps for the manufacture of the porous inorganic particulate materials according to the present invention include an optional rinsing step, e.g. with water, a drying step, and a calcining step. Typically drying is achieved at temperatures between 20 and 80°C; this step can be facilitated using an oven with air circulation or by applying reduced pressure.

Calcining is typically done at final temperatures between 400 and 900°C for one to several hours. The final temperature is reached using a temperature program, typically rising the temperature between 50 and 200°C per hour. Alternatively, in order to obtain hybridic materials, the calcining step can also be realized at lower temperatures, i.e. at final temperatures of maximum 350°C. A person skilled in the art can easily select the appropriate conditions for each case.

The pore size of macropores is determined using mercury porosimetry. It is also possible to estimate the pore dimensions from scanning electron micrographs (SEM). The pore size of mesopores and their specific surface area are determined using nitrogen adsorption/ desorption measurements (BET-method) which are performed by following standard protocols.

The disclosure is also directed to an inorganic particulate material mainly composed of silicon oxide, wherein the particulate material comprises macropores and mesopores, wherein the macropores have a mean diameter > 0.1 µm and the mesopores have a mean diameter between 2 and 100 nm, obtainable by the process of the present invention as described herein.

The particulate material obtainable by the process has a mean diameter from about 1 µm to about 2000 µm, preferably from about 1 µm to 1000 µm and more preferably from about 1 µm to 500 µm.

Further, one preferred embodiment is directed to such mesoporous particulate material, wherein said material has an irregular non-spherical shape.

In another embodiment, the particular material can be further surface modified by coating the surface of the constituent material with organic polymers to provide hydrophobic surface properties to the silicon oxide based material.
Organic polymers which are suitable for coating of the silicon oxide-based materials are organic materials which can be applied to the silicon oxide-based material as oligomer and/or polymer or organic oligomers and/or monomers which are applied to the silicon oxide-based material by polymerisation or polycondensation. The organic polymers can be chemi-or physisorbed on the silicon oxide-based material.
Suitable organic polymers which can be used to prepare the silicon oxide-based composite material are, for example, polystyrenes, polymethacrylates, polysiloxanes and derivatives thereof or copolymers of two or more suitable compounds, such as, for example, a coating of tetraalkoxysilane and methyltrialkoxysilane. Preference is given to chemi-or physisorbed polystyrenes, physisorbed poly(meth)acrylates or poly(meth)acrylic acid derivatives, such as, for example, poly(methacrylate), poly(2-hydroxyethyl methacrylate), a copolymer of 2-hydroxyethyl methacrylate and ethyl methacrylate or poly(octadecyl methacrylate) and silanes, which are especially preferred.
Coating process which can be used to prepare the silicon oxide-based composite material can take place by
- polymerisation or polycondensation of physisorbed monomers and/or oligomers without formation of covalent bonds to the silicon oxide-based material,
- polymerisation or polycondensation of physisorbed monomers and/or oligomers with formation of covalent bonds to the silicon oxide-based material,
- immobilisation (physisorption) of prepolymers without formation of bonds to the silicon oxide-based material or
- chemisorption of prepolymers on the silicon oxide-based material.

A solution which is employed for the coating of the silicon oxide-based material accordingly comprises either organic prepolymers or monomers and/or oligomers. In addition, it typically comprises a suitable solvent and optional further constituents, such as, for example, free-radical initiators. It is referred to in accordance with the invention as coating solution. Prepolymers here means that use is made of already oligomerised and/or polymerised compounds which, after introduction into the silicon oxide-based material, are not subjected to any further polymerisation reaction, i.e. are not cross-linked further with one another. Depending on the nature of the application, they are adsorbed onto the silicon oxide-based material (physisorption) or covalently bonded to the silicon oxide-based material (chemisorption).
By contrast, monomers and/or oligomers are compounds which are suitable for polymerisation or polycondensation and which are crosslinked or polymerised further by polymerisation of polycondensation after introduction into the silicon oxide-based material. Oligomers here are compounds which have already been generated in advance by crosslinking or polymerisation of monomers.
Processes for providing composite silicon oxide-based material by coating are known to the person skilled in the art and described, for example, in Handbuch der HPLC [Handbook of HPLC], Ed. K. K. Unger; GIT-Verlag (1989) and Porous Silica, K. K. Unger, Elsevier Scientific Publishing Company (1979).

One process for the coating of particles includes the application of a polymer solution or a solution of monomer and free-radical initiator. The solvent is subsequently removed.
According to a preferred embodiment the composite silicon-based material used for the modified release of biologically active agents is provided by reaction of the amorphous silicon oxide material with a silane compound capable of forming a covalent bond with a silanol group of the amorphous silicon oxide material.
Examples of the silane compound capable of forming a covalent bond by being reacted with a silanol group of the amorphous silicon oxide material include silazane, siloxane, or alkoxysilane, and partial hydrolysates of silazane, siloxane, or alkoxysilane, or oligomers such as a polymerized dimmer to pentamer of silazane, siloxane or cyclic-siloxane, alkoxysilane. Examples of the silazane include hexamethyldisilazane and hexaethyldisilazane.
Examples of the siloxane include hexamethyldisiloxane, 1,3-dibutyltetramethyldisiloxane, 1,3-diphenyltetramethyldisiloxane, 1,3-divinyltetramethyldisiloxane, hexaethyldisiloxane and 3-glycidoxypropylpentamethyldisiloxane.
Examples of the alkoxysilane include, for example, trimethylmethoxysilane, trimethylethoxysilane, trimethylpropoxysilane, phenyldimethylmethoxysilane, chloropropyldimethylmethoxysilane, dimethyldimethoxysilane, methyltrimethoxysilane, tetramethoxysilane, tetraethoxysilane, tetrapropoxysilane, tetrabutoxysilane, ethyltrimethoxysilane, dimethyldiethoxysilane, propyltriethoxysilane, n-butyltrimethoxysilane, n-hexyltrimethoxysilane, n-octyltriethoxysilane, n-octylmethyldiethoxysilane, n-octadecyltrimethoxysilane, phenyltrimethoxysilane, phenylmethyldimethoxysilane, phenetyltrimethoxysilane, dodecyltrimethoxysilane, n-octadecyltriethoxysilane, phenyltrimethoxysilane, diphenyldimethoxysilane. These silane compounds may be used individually or in combination of two or more types thereof. Silane compounds having reactive groups capable of bonding colloidal silica particles with the polymer while curing the curable composition of the present invention can enhance the properties of the cured article, so that such silane compounds are preferred.
According to a preferred embodiment of the invention the composite silicon oxide-based material used for the modified release of the biologically active agent is modified by reaction with a compound having the formula (I)

SiXₙR¹₍₃₋ₙ₎R² (I)

wherein
- X: is a reactive group,
- R¹: is C1-C5 alkyl,
- n: is 1, 2 or 3; and
- R²: is unsubstituted or substituted alkyl or aryl.
Therefore, one preferred object of the present invention is directed to the use of composite silicon oxide-based material for the modified release of biologically active agents, which is modified by reaction with a compound having the formula (I)

SiXₙR¹₍₃₋ₙ)R² (I)

wherein
- X: is a reactive group,
- R¹: is C₁-C₅ alkyl,
- n: is 1, 2 or 3; and
- R²: is unsubstituted or substituted alkyl or aryl.
X can be C₁-C₃ alkoxy, preferably methoxy or ethoxy or a halogen such as F, Cl, Br or J, preferably Cl.
In R² alkyl can be unbranched or branched alkyl having 1 to 20 C atoms, which optionally may be substituted by 1, 2, 3 or 4 OH, Diol, NH₂, Epoxy and/or CN whereby unbranched alkyl is preferred. Examples of suitable alkyl are methyl, ethyl, n-propyl, n-butyl, n-pentyl, n-hexyl, n-octyl, n-decyl, n-dodecyl or n-octadecyl, whereby n-octyl and n-octadecyl are preferred. Aryl can be phenyl or phenylalkyl such as, for example phenylmethyl, phenylethyl, phenylpropyl or phenylbutyl, whereby phenylbutyl is preferred.

According to a particularly preferred embodiment in the silane compound of formula (I) used for the modification independendly from each other
X is methoxy, ethoxy or halogen,
R2 is n-octyl, n-octadecyl, methyl, ethyl, n-propyl, isopropyl, n-butyl, isobutyl, tert.-butyl or phenylbutyl.
Therefore, one particularly preferred object of the invention is directed to the use of composite silicon oxide-based material for the modified release of biologically active agents, which is modified with a silane compound of formula (I),
wherein independendly from each other
X is methoxy, ethoxy or halogen,
R2 is n-octadecyl, n-octyl, methyl, ethyl, n-propyl, isopropyl, n-butyl, isobutyl, tert.-butyl or phenylbutyl.

Advantageously the material provided by the present invention can be used as matrix forming agent in a modified release delivery system for a bioactive agent. Therefore, the disclosure is also directed to a modified release delivery system comprising a bioactive agent and inorganic mesoporous and macroporous particulate material as it is obtainable according to the process of the present invention.

A bioactive agent, which can be present in the modified release system can be any chemical substance or protein, which are capable of providing a local or systemic biological, physiological, or therapeutic effect in the subject to which it is applied. Preferred examples of a bioactive agent are pharmaceutical drugs, biological macromolecules, vaccines, vitamins or minerals. In terms of its activity the bioactive agent, which can be present in the modified release system, can be, for example an agent that act to control or prevent infection or inflammation, enhance cell growth and tissue regeneration, control tumor growth, act as an analgesic, promote anti-cell attachment or enhance bone growth, among other functions. Other suitable bioactive agents can include anti-viral agents, hormones, antibodies, or therapeutic proteins. Still other bioactive agents include prodrugs, which are agents that are not biologically active when administered but upon administration to a subject are converted to bioactive agents through metabolism or some other mechanism. Further suitable bioactive agents are vaccines, i.e. substances used to stimulate the production of antibodies and provide immunity against one or several diseases, prepared from the causative agent of a disease, its products, or a synthetic substitute, treated to act as an antigen without inducing the disease.

According to a preferred embodiment the modified release delivery system contains a pharmaceutical drug. Therefore, one embodiment is directed to a modified release delivery system, wherein the bioactive agent is a pharmaceutical drug.

According to a further preferred embodiment the modified release delivery system contains a vaccine. Therefore, one embodiment is directed to a modified release delivery system, wherein the bioactive agent is a vaccine.

As used herein, the term "modified release" means that the release of the bioactive agent from the delivery system or a portion thereof upon contact of the dosage form or portion thereof with a liquid medium is different to the release of the same bioactive agent from a conventional immediate release formulation, wherein the release is mainly controlled by the solubility of the bioactive agent in the liquid medium. Accordingly, modified release includes, but is not limited to accelerated release (i.e. an increased dissolution rate), sustained-release, extended release, slow release, delayed release and the like.

The inorganic particulate material comprising macropores and mesopores as it is obtainable by the process of the present invention is especially usable to increase the dissolution of bioactive agents especially suitable to increase the dissolution rate of poorly water-soluble or water-insoluble bioactive agents. Poorly water-soluble substances are understood to have a solubility in water of < 10 mg/ml, in particular < 5 mg/ml and more particularly < 1 mg/ml, practically water-insoluble or insoluble substances are those having a solubility in water of < 0.1 mg/ml. The term "water-solubility" or "solubility in water" in the present application refers to the respective solubility measured at 25°Celsius.

Accordingly one further object is directed to a modified release system comprising a bioactive agent and inorganic mesoporous and macroporous particulate material as it is obtainable by the process according to the present invention, wherein the bioactive agent has a water-solubility of < about 10 mg/ml, preferably from about 0.1 mg/ml to about 5 mg/ml and more preferably from about < 1 mg/ml.

In another embodiment, the inorganic particulate material comprising macropores and mesopores as it is obtainable by the process of the present invention can also be used as as carrier for liquids (i.e. liquid pharmaceutical formulations or liquid APIs). In this case, the bioactive agent itself may be liquid or may be dissolved or formulated in a liquid formulation (e.g. oily, lipids, aqueous formulations).
Accordingly one further object is directed to a modified release system comprising a bioactive agent and inorganic mesoporous and macroporous particulate material as it is obtainable by the process according to the present invention, wherein the bioactive agent is present in a liquid form.

The modified release system contains the bioactive agent in an amount of from about 0.1 to about 90% by weight, preferably from about 0.2 to about 75% by weight, more preferably from about 5 to about 40% by weight most preferably from about 10 to about 30% by weight. Thus the description also discloses a modified release system as described herein, wherein the bioactive agent is present in an amount of from about 0.1 to about 90% by weight, preferably from about 0.2 to about 75% by weight, more preferably from about 5 to about 40% by weight most preferably from about 10 to about 30% by weight.

The bioactive agent can be applied to the inorganic particulate material by using the loading techniques known in the art, as, for example, by adsorption from a solution of the bioactive agent in a suitable solvent to the inorganic material and subsequent separation, by wetness impregnation of the inorganic material with a concentrated solution of the bioactive agent in a suitable solvent such as, for example, ethanol, CH₂Cl₂ or acetone and subsequent solvent evaporation, by spray-drying of a mixture of bioactive agent in a suitable solvent, by heating of a mixture of the bioactive agent and the particulate material or by drug loading with supercritical fluids. The modified release system can be formulated as an oral, a topical or a parenteral administration form, preferably as an oral administration form. Consequently, the invention is further directed to the use of the modified release system as described herein, wherein said system is an oral or a topical or a parenteral administration form.

Suitable for oral administration forms include tablets, capsules, powders, dragees, suspensions; suitable topical administration forms include ointments, creams, suspensions, lotions, powders, solutions, pastes, gels, sprays, aerosols or oils.

If an oral administration form is used, tablets, capsules and powders are preferred, if a topical administration form is used, ointments, creams, suspensions and powders are preferred. Accordingly, disclosed is also a modified release system as described herein, wherein said release system is an oral application form, which is a tablet, a capsule, a powder, or a dragee, or a topical administration form, which is an ointment, a cream, a suspension or a powder and a parenteral administration form, which comprises microparticles or is an implant.

The modified release system is suitable to be used for the administration of at least one bioactive agent to a biological organism, preferably to a mammal, more preferably to a human. Accordingly, discloses is also the use of the modified release system as described herein for the administration of at least one bioactive agent to a mammal, preferably to a human.

The application forms described above are well known in the art. For example, if the modified release system is in the form of a tablet or capsule, the bioactive agent loaded inorganic material can be combined with an oral, non-toxic and pharmaceutically acceptable inert excipient, such as, for example, ethanol, glycerol, water and the like. Powders can be composed of the bioactive agent loaded inorganic material itself, which may be further comminuted, or can be prepared, for example, by mixing the bioactive agent loaded inorganic, which may have been comminuted, with a comminuted pharmaceutical excipient, such as, for example, an edible carbohydrate, such as, for example, starch or mannitol. A flavour, preservative, dispersant and dye may likewise be present.

Capsules can be produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the active agent loaded in a inorganic, which may have been comminuted in a suitable manner, with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape which are broken up to form granules. The granules can be lubricated by addition of stearic acid, a stearate salt, talc or mineral oil in order to prevent sticking to the tablet casting moulds. The lubricated mixture is then pressed to give tablets. The bioactive agent loaded inorganic material can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

For the treatment of external tissue, for example mouth and skin, the formulations are preferably applied as topical ointment or cream. In the case of formulation to give an ointment, the bioactive agent loaded inorganic material can be employed either with a paraffinic or a water-miscible cream base. Alternatively, the bioactive agent loaded inorganic material can be formulated to give a cream with an oil-in-water cream base or a water-in-oil base.

The term "implant" denotes a solid modified release delivery system, which is deposited in any suitable body tissues or cavities to form a reservoir or pool which slowly migrates to surrounding tissues and organs and eventually becomes systemically distributed. However, these distinctions are not always rigidly adhered to in the art, and consequently, it is contemplated that there is included within the scope of the present invention liquid implants and solid depots, and even mixed solid and liquid forms for each.
Also included are implants which are placed beneath the epidermal layer of the skin, i. e. between the epidermis and the dermis of the skin of the patient being treated. Such an implant will be formulated in accordance with well known principles and materials commonly used in this delivery technology, and may be prepared in such a way as to provide controlled-, sustained-, and/or delayed-release of the active ingredient into the systemic circulation of the patient. Advantageously such type of implant can be easily administered and removed by minimal incision or can be applied by using an implant syringe, which are well known in the art for such purpose.
The solid modified release delivery system can be also parenterally applied in the form of microparticles via a syringe using a carrier liquid. Preferably, the microparticles are mixed with the carrier liquid to be injected into a patient. In one embodiment the microparticles mixed with an organic solvent to produce a liquid or gel which may be injected into a patient.

Further formulations adapted for topical application in the mouth encompass lozenges, pastilles and mouthwashes.

The following examples explain the invention without being restricted thereto.

### Examples:

### Example 1:

In a 1-L Schott glass bottle, 71.05g of polyethylene glycol 10,000 and 63.00g of urea are dissolved in 700 ml of 0.01M acetic acid via the use of a magnetic stir bar. Once the polyethylene glycol and the urea are completely dissolved (8-15 minutes), the solution is then transferred to a 1-L, double jacketed, 3-necked reactor which has previous been cooled to 3°C with a low temperature thermostat (Lauda ECO 415 S). The reactor is equipped with an overhead stirrer (Heidolph Rührwerk RZR 2102 Control) and a multi-stir blade, with the blades configured at 3 different heights. The overhead stirrer is zeroed, with respect to torque, per the manufacturer's instructions prior to the start of the process. The solution is stirred at 250 rpm and after about 45 minutes cooled to a temperature of 5°C. Once this temperature is reached, the stirring is discontinued and 366.10g of tetramethyl siloxane is added to the solution, creating the reaction mixture. At this point, stirring is continued at 250 rpm. After 30 minutes stirring, the temperature of the low temperature thermostat controlling the reaction mixture temperature is increased to 30°C and the reaction mixture is stirred for 50 minutes more. Once the torque on the overhead stirrer reaches 3.1 Ncm, the stirring rate is increased to 500 rpm. After 5 minutes stirring at 500 rpm, the stir speed is increased to 600 rpm. After a further 40 minutes stirring at this speed, the speed is decreased to 250 rpm and the temperature of the low temperature thermostat is set at 80°C. The suspension is then stirred at this speed and temperature for 5 hours. At the end of 5 hours, the reaction mixture is cooled to <40°C and the resulting suspension is discharged from the reactor into a 2-L beaker. The particulate silica material is separated from the mother liquor utilizing vacuum filtration and rinsed first with water and then with methanol. After the majority of the methanol is removed, the remaining silica powder is white in color and freely flowable. The silica powder is placed in a porcelain dish and dried for 4 hours in a vacuum drying oven set at 100°C. After that the silica powder is sieved over a 200µm metal sieve. The material is then calcinated for 5 hours at 600°C to remove any organic material.
The calcined silca gel is analyzed for surface area and mesopore size using nitrogen adsorption-desorption methods, macropore size is measured via mercury intrusion porosimetry, particle size distribution via laser light scattering. The mesopore size is determined using the BJH desorption method.

Particle measurement of this and all other Examples is performed using the following Instruments:
- Hg-Intrusion: PoreMaster 60 from Quantachrome Instruments, 1900 Corporate Drive Boynton Beach, Florida 33426 USA;
- BET: Accelerated Surface Area and Porosimetry System ASAP® 2420 from Micromeritics Instrument Corporation, 4356 Communications Drive, Norcross, GA 30093-2901, USA;
- Malvern Mastersizer 2000 from Malvern Instruments Ltd, Enigma Business Park, Grovewood Road, Malvern, Worcestershire WR14 1XZ, United Kingdom.

### Results:

Nitrogen Adsorption/Desorption
Surface Area: 743.2 m2/g
Pore Volume: 0.80 cm3/g
Mesopore Size: 40.6 Å

### Mercury Porosimetry:

Macropore Size: 6.1 µm

### Particle Size Distribution

d(10) - 5.1 µm
d(50) - 15.8 µm
d(90) - 34.4 µm

### Example 2: Lower Torque Method

In a 1-L Schott glass bottle, 71.05g of polyethylene glycol 10,000 and 63.00g of urea are dissolved in 700 ml of 0.01M acetic acid via the use of a magnetic stir bar. Once the polyethylene glycol and the urea are completely dissolved (8-15 minutes), the solution is then transferred to a 1-L, double jacketed, 3-necked reactor which has previous been cooled to 3°C with a low temperature thermostat (Lauda ECO 415 S). The reactor is equipped with an overhead stirrer (Heidolph Rührwerk RZR 2102 Control) and a multi-stir blade, with the blades configured at 3 different heights. The overhead stirrer is zeroed, with respect to torque, per the manufacturer's instructions prior to the start of the process. The solution is stirred at 250 rpm and after about 45 minutes cooled to a temperature of 5°C. Once this temperature is reached, the stirring is discontinued and 366.10g of tetramethyl siloxane is added to the solution, creating the reaction mixture. At this point, stirring is continued at 250 rpm. After 30 minutes stirring, the temperature of the low temperature thermostat controlling the reaction mixture temperature is increased to 30°C and the reaction mixture is stirred for 50 minutes more. Once the torque on the overhead stirrer reaches 2.0 Ncm, the stirring rate is increased to 500 rpm. After 5 minutes stirring at 500 rpm, the stir speed is increased to 600 rpm. After a further 40 minutes stirring at this speed, the speed is decreased to 250 rpm and the temperature of the low temperature thermostat is set at 80°C. The suspension is then stirred at this speed and temperature for 5 hours. At the end of 5 hours, the reaction mixture is cooled to <40°C and the resulting suspension was discharged from the reactor into a 2-L beaker. The particulate silica material is separated from the mother liquor utilizing vacuum filtration and rinsed first with water and then with methanol. After the majority of the methanol is removed, the remaining silica powder is white in color and freely flowable. The silica powder is placed in a porcelain dish and dried for 4 hours in a vacuum drying oven set at 100°C. After that the silica powder is sieved over a 200µm metal sieve. The material is then calcinated for 5 hours at 600°C to remove any organic material.

The calcined silca gel is analyzed for surface area and mesopore size using nitrogen adsorption-desorption methods, macropore size was measured via mercury intrusion porosimetry, particle size distribution via laser light scattering.

### Results:

Nitrogen Adsorption/Desorption
Surface Area: 795.6 m2/g
Pore Volume: 0.86 cm3/g
Mesopore Size: 40.7 Å

### Mercury Porosimetry:

Macropore Size: 2.3 µm

### Particle Size Distribution

d(10) - 3.5 µm
d(50) - 7.6 µm
d(90) - 20.8 µm

### Example 3: Higher Torque Method

In a 1-L Schott glass bottle, 71.05g of polyethylene glycol 10,000 and 63.00g of urea are dissolved in 700 ml of 0.01M acetic acid via the use of a magnetic stir bar. Once the polyethylene glycol and the urea are completely dissolved (8-15 minutes), the solution is then transferred to a 1-L, double jacketed, 3-necked reactor which has previous been cooled to 3°C with a low temperature thermostat (Lauda ECO 415 S). The reactor is equipped with an overhead stirrer (Heidolph Rührwerk RZR 2102 Control) and a multi-stir blade, with the blades configured at 3 different heights. The overhead stirrer is zeroed, with respect to torque, per the manufacturer's instructions prior to the start of the process. The solution is stirred at 250 rpm and after about 45 minutes cooled to a temperature of 5°C. Once this temperature is reached, the stirring is discontinued and 366.10g of tetramethyl siloxane is added to the solution, creating the reaction mixture. At this point, stirring is continued at 250 rpm. After 30 minutes stirring, the temperature of the low temperature themostat controlling the reaction mixture temperature is increased to 30°C and the reaction mixture is stirred for 50 minutes more. Once the torque on the overhead stirrer reaches 4.5 Ncm, the stirring rate is increased to 500 rpm. After 5 minutes stirring at 500 rpm, the stir speed is increased to 600 rpm. After a further 40 minutes stirring at this speed, the speed is decreased to 250 rpm and the temperature of the low temperature thermostat is set at 80°C. The suspension is then stirred at this speed and temperature for 5 hours. At the end of 5 hours, the reaction mixture is cooled to <40°C and the resulting suspension is discharged from the reactor into a 2-L beaker. The particulate silica material is separated from the mother liquor utilizing vacuum filtration and rinsed first with water and then with methanol. After the majority of the methanol is removed, the remaining silica powder is white in color and freely flowable. The silica powder is placed in a porcelain dish and dried for 4 hours in a vacuum drying oven set at 100°C. After that the silica powder is sieved over a 200µm metal sieve. The material is then calcinated for 5 hours at 600°C to remove any organic material.

The calcined silca gel is analyzed for surface area and mesopore size using nitrogen adsorption-desorption methods, macropore size is measured via mercury intrusion porosimetry, particle size distribution via laser light scattering.

### Results:

Nitrogen Adsorption/Desorption
Surface Area: 876.8 m2/g
Pore Volume: 0.92 cm3/g
Mesopore Size: 39.3 Å

### Mercury Porosimetry:

Macropore Size: 6.9 µm

### Particle Size Distribution

d(10) - 5.4 µm
d(50) - 18.0 µm
d(90) - 42.6 µm

### Example 4: Comparative Example: Use of visual indication of phase separation

In a 1-L Schott glass bottle, 71.05g of polyethylene glycol 10,000 and 63.00g of urea are dissolved in 700 ml of 0.01M acetic acid via the use of a magnetic stir bar. Once the polyethylene glycol and the urea are completely dissolved (8-15 minutes), the solution is then transferred to a 1-L, double jacketed, 3-necked reactor which has previous been cooled to 3°C with a low temperature thermostat (Lauda ECO 415 S). The reactor is equipped with an overhead stirrer (Heidolph Rührwerk RZR 2102 Control) and a multi-stir blade, with the blades configured at 3 different heights. The solution is stirred at 250 rpm and after about 51 minutes cooled to a temperature of 5°C. Once this temperature is reached, the stirring is discontinued and 366.08 g of tetramethyl siloxane is added to the solution, creating the reaction mixture. At this point, stirring is continued at 250 rpm. After 30 minutes stirring, the temperature of the low temperature thermostat controlling the reaction mixture temperature is increased to 30°C and the reaction mixture is stirred for 50 minutes more. The stirring is then stopped and phase separation is determined to occur after about 32 minutes. After about 15 minutes after the notice of the phase separation, the stirring is begun again at a rate of 250 rpm. After a further 5 minutes, the stirring speed is increased to 500 rpm and after a further 5 minutes, the stirring is increased to 600 rpm. After a further 40 minutes stirring at this speed, the speed is decreased to 250 rpm and the temperature of the low temperature thermostat is set at 80°C. The suspension is then stirred at this speed and temperature for 5 hours. At the end of 5 hours, the reaction mixture is cooled to <40°C and the resulting suspension is discharged from the reactor into a 2-L beaker. The particulate silica material is separated from the mother liquor utilizing vacuum filtration and rinsed first with water and then with methanol. After the majority of the methanol is removed, the remaining silica powder is white in color and freely flowable. The silica powder is placed in a porcelain dish and dried for 4 hours in a vacuum drying oven set at 100°C. After that the silica powder is sieved over a 200µm metal sieve. The material is then calcinated for 5 hours at 600°C to remove any organic material.

The calcined silca gel is analyzed for surface area and mesopore size using nitrogen adsorption-desorption methods, macropore size is measured via mercury intrusion porosimetry, particle size distribution via laser light scattering.

### Results:

Nitrogen Adsorption/Desorption
Surface Area: 749.8 m2/g
Pore Volume: 0.73 cm3/g
Mesopore Size: 37.7 Å

### Mercury Porosimetry:

Macropore Size: 4.8 µm

### Particle Size Distribution

d(10) - 5.8 µm
d(50) - 20.8 µm
d(90) - 53.4 µm

### Example 5: Drug Loading

Fenofibrate, a synthetic compound mainly used to reduce cholesterol levels, which is poorly soluble in aqueous solutions (0.3 µg/ml in water; see Vogt, M. et al., Eur J Pharm Biopharm 68 (2) (2008) 283 - 288), is used as model drug.
The silica material of the present invention is drug loaded with fenofibrate by using wetness impregnation. For this purpose 10.74 g of fenofibrate is dissolved in 40 mL of acetone at room temperature. A 400 ml beaker (heated by a water bath at 60°C; equipped with an overhead stirrer and paddle) is filled with 25.02 g of silica material synthesized in accordance to Example 1. The fenofibrate solution is drawn into 30 ml syringe and connected to PEEK tubing through the use of a Luer-lock system. The syringe is then placed in a syrine pump (Harvard Apparatus PHD Ultra) and the infusion is begun at a rate of 0.75 ml/min. During the infusion, the end of the PEEK tubing is placed just above the surface of the silica in the beaker, which during the loading is being stirred at a rate of 15 rpm through the use of an overhead motor (Heidolph Rührwerk RZR 2102 Control) and a PTFE stirrer that is cut to match the inner diameter of the beaker. The beaker is covered with a thin film of Parafilm and has three ports fashioned to accommodate: (1) the PEEK tubing to deliver the fenofibrate solution, (2) a tube delivering a slight stream of nitrogen over the product, and (3) a vacuum line used to promote the evaporation of the acetone from the silica. The procedure of impregnating and subsequently evaporating is repeated until the entire fenofibrate solution is evaporated. Additionally, the obtained powder is dried under vacuum at 50°C over night. The resulting drug load is 30% by weight.
The dissolution rates of fenofibrate loaded formulation prepared as set forth above and pure crystalline fenofibrate are tested using USP Apparatus II (rotating paddle) dissolution tester with on-line UV sampler and measurement system (dissolution system from Sotax: Sotax AT 7 smart on/offline with Agilent photometer 8453) (conditions: simulated gastric fluid (SGF) without pepsin; 1000 mL vessel; 37°C; 75 rpm; 0.1% sodium dodecylsulfate (SDS)). For comparison proposes the same dissolution test is performed with the pure crystalline fenofibrate.
The fenofibrate loaded samples tested contain 50 mg of fenofibrate which is confirmed by high performance liquid chromatography (HPLC) with UV detector, pure crystalline fenofibrate is tested in the same amount (50 mg).

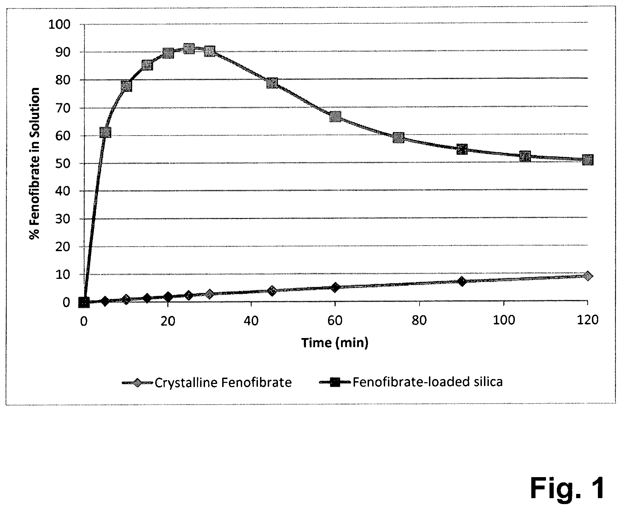

Figure 1 summarizes the dissolution rates of the samples tested.
For evaluation of success of loading XRD (STOE & CIE GmbH Stoe StadiP 611 KL) and DSC (Mettler Toledo DSC 821e Differential Scanning Calorimeter Thermal Analyzer) measurements are performed. The results confirm that the used API deposits in an amorphous form onto the surface of the carrier.
The resulting drug load of 30% by weight can be double checked by NMR (Bruker Avance 500 NMR Spectrometer with cryoprobe).

## Claims

1. A process for producing inorganic particulate material mainly composed of silicon oxide, wherein the particulate material comprises mesopores and macropores and the process includes the steps of:
(a) dissolving a water-soluble polymer or another pore forming agent and a precursor for a matrix dissolving agent in a medium that promotes the hydrolysis of the metalorganic compound (see step b);
(b) mixing a metalorganic compound or a mixture of metalorganic compounds which contains hydrolyzable ligands to promote hydrolysis reaction;
(c) solidifying the mixture through a sol-gel transition, wherein the mixture is continuously stirred, whereby particles are prepared which have three dimensional interconnected phase domains one rich in solvent the other rich in inorganic component in which surface pores are contained, wherein in step (c) changes in torque are monitored;
(d) setting the matrix dissolving agent free from its precursor, whereby the matrix dissolving agent modifies the structure of said inorganic component;
(e) removing the solvent by evaporation drying and/or heat-treatment.

2. Process according to claim 1, wherein in step (c) the size of the particles is controlled by adjusting the stirring speed over the reaction time.

3. Process according to claim 1 or 2 further including the step (f) calcining the particles.

4. Process according to one or more of claims 1 to 3, wherein the macropores have a mean diameter > 0.1 µm and the mesopores have a mean diameter between 2 and 100 nm.

5. Process according to one or more of claims 1 to 4, wherein said precursor of the matrix dissolving agent is a compound having an amido group or an alkylamido group, preferably urea.

6. Process according to one or more of claims 1 to 5, wherein the pore forming agent is a non-ionic surfactant.

## Patentansprüche

1. Verfahren zur Herstellung von anorganischem Teilchenmaterial, das überwiegend aus Siliciumoxid besteht, bei dem das Teilchenmaterial Mesoporen und Makroporen enthält und das Verfahren die folgenden Schritte umfasst:
(a) Lösen eines wasserlöslichen Polymers oder eines anderen porenbildenden Mittels und eines Vorläufers für ein Matrixlösemittel in einem Medium, das die Hydrolyse der Organometallverbindung fördert (siehe Schritt b);
(b) Mischen einer Organometallverbindung oder eines Gemisches von Organometallverbindungen, die/das hydrolysierbare Liganden zur Förderung der Hydrolysereaktion enthält;
(c) Verfestigen des Gemisches durch einen Sol-Gel-Übergang, bei dem das Gemisch ständig gerührt wird, wodurch Teilchen hergestellt werden, die dreidimensionale miteinander verbundene Phasendomänen aufweisen, eine reich an Lösungsmittel, die andere reich an anorganischer Komponente, worin Oberflächenporen enthalten sind, bei dem in Schritt (c) Drehmomentänderungen überwacht werden;
(d) Freisetzen des Matrixlösemittels aus seinem Vorläufer, wodurch das Matrixlösemittel die Struktur der anorganischen Komponente modifiziert;
(e) Entfernen des Lösungsmittels durch Verdampfungstrocknung und/oder Wärmebehandlung.

2. Verfahren nach Anspruch 1, bei dem in Schritt (c) die Größe der Teilchen durch Einstellen der Rührgeschwindigkeit im Verlauf der Reaktion gesteuert wird.

3. Verfahren nach Anspruch 1 oder 2, weiterhin enthaltend den Schritt (f) Kalzinieren der Teilchen.

4. Verfahren nach einem oder mehreren der Ansprüche 1 bis 3, bei dem die Makroporen einen mittleren Durchmesser > 0,1 µm aufweisen und die Mesoporen einen mittleren Durchmesser zwischen 2 und 100 nm aufweisen.

5. Verfahren nach einem oder mehreren der Ansprüche 1 bis 4, bei dem es sich bei dem Vorläufer des Matrixlösemittels um eine Verbindung mit einer Amidogruppe oder einer Alkylamidogruppe, vorzugsweise Harnstoff, handelt.

6. Verfahren nach einem oder mehreren der Ansprüche 1 bis 5, bei dem es sich bei dem Porenbildner um ein nichtionisches Tensid handelt.

## Revendications

1. Procédé pour produire un matériau particulaire inorganique qui est essentiellement composé d'oxyde de silicium, dans lequel le matériau particulaire comprend des mésopores et des macropores et le procédé inclut les étapes constituées par :
(a) la dissolution d'un polymère soluble à l'eau ou d'un autre agent de formation de pores et d'un précurseur pour un agent de dissolution de matrice dans un milieu qui favorise l'hydrolyse du composé organométallique (voir l'étape b) ;
(b) le mélange d'un composé organométallique ou d'un mélange de composés organométalliques qui contient des ligands pouvant être hydrolysés de manière à favoriser la réaction d'hydrolyse ;
(c) la solidification du mélange par l'intermédiaire d'une transition sol-gel, dans lequel le mélange est agité en continu, d'où il résulte que des particules sont préparées, lesquelles comportent des domaines de phase interconnectés tridimensionnels dont l'un est riche en solvant et dont l'autre est riche en composant inorganique où des pores de surface sont contenus, dans lequel, au niveau de l'étape (c), des modifications de couple sont surveillées ;
(d) le fait de faire en sorte que l'agent de dissolution de matrice soit libéré de son précurseur, d'où il résulte que l'agent de dissolution de matrice modifie la structure dudit composant inorganique ; et
(e) l'évacuation du solvant par séchage à évaporation et/ou traitement thermique.

2. Procédé selon la revendication 1, dans lequel, au niveau de l'étape (c), la taille des particules est contrôlée en réglant la vitesse d'agitation au fil du temps de la réaction.

3. Procédé selon la revendication 1 ou 2, incluant en outre l'étape (f) de calcination des particules.

4. Procédé selon une ou plusieurs des revendications 1 à 3, dans lequel les macropores présentent un diamètre moyen > 0,1 µm et les mésopores présentent un diamètre moyen entre 2 et 100 nm.

5. Procédé selon une ou plusieurs des revendications 1 à 4, dans lequel ledit précurseur de l'agent de dissolution de matrice est un composé qui comporte un groupe amido ou un groupe alkylamido, de préférence de l'urée.

6. Procédé selon une ou plusieurs des revendications 1 à 5, dans lequel l'agent de formation de pores est un agent tensioactif non ionique.
